# EUROPEAN PATENT APPLICATION

(11) **EP 4 052 728 A1**
(43) Date of publication of application: **07.09.2022**
(21) Application number: 20883649.4
(22) Date of filing: 30.10.2020
(51) Int. Cl.: A61K 45/00, A61P 17/00, A61Q 19/00, A61K 9/08, A61K 9/12, A61K 8/02, A61K 8/19, A61K 8/60, A61K 47/02, A61K 31/7076

(54) **METHOD FOR USING ULTRAFINE BUBBLES**

(30) Priority: 01.11.2019 JP 2019200313
(71) Applicant: Shiseido Company, Ltd., Chuo-ku Tokyo 104-0061 (JP)
(72) Inventor: KINOSHITA, Koichi, Tokyo 104-0061 (JP); YAMASHITA, Takahiro, Tokyo 104-0061 (JP); TAKECHI, Miwa, Tokyo 104-0061 (JP)
(74) Representative: Ter Meer Steinmeister & Partner
(86) International application number: PCT/JP2020/040945
(87) International publication number: WO 2021/085633

(57) **Abstract**

[Objective]

To improve the permeability of external skin agents such as cosmetics into skin.

[Constitution]

In the case that an external skin agent that contains a specific component is to be applied to the skin of a right hand (1R), a liquid that contains ultrafine bubbles is applied to the portion of the skin onto which the external skin agent is to be applied. The application of the liquid is performed prior to the external skin agent being applied, by spraying the liquid from an aerosol container (10) having a spray actuator (11) with a flow rate adjusting mechanism.

## Description

### Technical Field

The present invention is related to a method of utilizing ultrafine bubbles.

### [Background Art]

Conventionally, a technique in which a liquid that contains extremely fine bubbles is caused to adhere on the surface of a human body by application etc., to realize a specific function unique to the type of liquid is known, as disclosed in Patent Documents 1 through 3, for example.

Specifically, Patent Document 1 discloses that plasma discharge processed water, which is a high concentration ozone water, may be utilized as a cosmetic liquid as is, or utilized as a raw material for various cosmetic liquids such as a cosmetic moisturizing liquid and a cosmetic milky lotion. In addition, Patent Document 1 discloses that the plasma discharge processed water exhibits superior permeability into skin as well as high moisturizing effects (refer to paragraphs 0040 and 0043). Further, Patent Document 1 discloses that a technique in which ozone is caused to be in a state of a great number of ultrafine bubbles called microbubbles, nanobubbles, etc., and blown into water is known as a method for obtaining the high concentration ozone water (refer to paragraph 0002).

In addition, Patent Document 2, which is related to a method for producing fine bubbles, discloses that the effects of utilizing hot water that contains a great number of fine bubbles include removal of dirt from within pores of the skin, as well as removal of dirt, oil, and fats from hair due to the high permeability thereof (refer to paragraph 0027). Patent Document 2 also discloses that hot water that exhibits the aforementioned effects is that in which fine bubbles having diameters of less than or equal to 500 nm, particularly diameters within a range from 100 to 300 nm, are generated, and that these types of fine bubbles encompass the concept of microbubbles, micro nanobubbles, and nanobubbles (refer to paragraphs 0030 and 0031).

Further, Patent Document 3 discloses a method for providing a dispersion that does not contain a surfactant within a mixture of a dispersed substance and a liquid dispersion medium by utilizing an ultrafine bubble generating apparatus. This method produces a composition that contains ultrafine bubbles having a modal particle diameter of less than or equal to 500 nm, a hydrophobic medicament, and water. Patent Document 3 also discloses that in such a composition, the effects of the medicament are more favorably expressed due to the ultrafine bubbles also being present. In the case that the medicament has transpiration properties, the transpiration performance improves. In the case that the medicament is an antifungal agent, etc., the permeability into the skin thereof improves (refer to paragraphs 0015 and 0016). Patent Document 3 lists various pharmaceutical preparations and cosmetics as examples of the medicament (refer to paragraph 0021).

Note that in Patent Documents 1 through 3, the terms microbubbles, micro nanobubbles, and nanobubbles are employed with respect to the fine bubbles or the ultrafine bubbles which are included in a liquid such as water. Currently, fine bubbles having particle diameters of less than or equal to 1000 nm are defined as "ultrafine bubbles" by ISO/TC281 (Fine Bubble Technology). The present invention is related to fine bubbles having particle diameters of less than or equal to 1000 nm, and therefore these fine bubbles will be referred to as "ultrafine bubbles".

Meanwhile, the apparatuses which are disclosed in Patent Document 3 are known as apparatuses that generate the aforementioned ultrafine bubbles within a liquid such as water. The apparatuses which are exemplified in Patent Document 3 are those that apply the static mixing method, the venturi method, the cavitation method, the vapor agglomeration method, the ultrasound method, the spiral flow method, the pressurized dissolving method, the fine pore method, and the gas liquid mixture shearing method. Further, the apparatuses which are disclosed in Patent Document 4 and Patent Document 5, for example, are also known. The apparatuses which are disclosed in these documents are equipped with a rotatable shaft, on the outer peripheral surface of which a plurality of pyramidal protrusions or rectangular blades are mounted, and a housing pipe such as a water pipe through which liquids are capable of flowing, the rotatable shaft being provided within the housing pipe.

### [Background Art Documents]

[Patent Document 1] Japanese Unexamined Patent Publication No. 2006-289236
[Patent Document 2] Japanese Unexamined Patent Publication No. 2010-201397
[Patent Document 3] Japanese Re-Publication of PCT International Application No. 2011/16529
[Patent Document 4] Japanese Patent No. 6077627
[Patent Document 5] Japanese Patent No. 6490317

### [Summary]

### [Technical Problem]

The objective of the present invention is to provide a method that improves the permeability of external skin agents into skin in cases that external skin agents such as cosmetics are applied onto the skin.

### [Solution to the Problem]

The present invention improves the permeability of external skin agents into skin by utilizing the aforementioned ultrafine bubbles. That is, the method of utilizing ultrafine bubbles according to the present invention applies a liquid that contains ultrafine bubbles onto a portion of the skin onto which an external skin agent that contains a specific component to the skin of the human body is to be applied, or applies an external skin agent that contains ultrafine bubbles onto the skin, in order to promote permeation of the specific component into the skin.

Note that it is desirable for the method of utilizing ultrafine bubbles according to the present invention to be applied in the case that the specific component is adenosine.

In addition, in the method of utilizing ultrafine bubbles according to the present invention, it is particularly desirable for the principal component of the liquid that contains ultrafine bubbles to be water. The expression "principal component" refers to the component having the greatest percentage by weight from among a plurality of components.

Further, examples of the external skin agent to be employed in the method of utilizing ultrafine bubbles according to the present invention include cosmetics and external pharmaceutical preparations, for example.

### [Effects of the Invention]

The method of utilizing ultrafine bubbles according to the present invention applies a liquid that contains ultrafine bubbles onto a portion of the skin onto which an external skin agent is to be applied to the skin, or applies an external skin agent that contains ultrafine bubbles onto the skin. Therefore, permeation of the specific component which is contained in the external skin agent into the skin is promoted by the function of the ultrafine bubbles.

### [Brief Description of the Drawings]

FIG. 1 is a schematic diagram that illustrates the method for utilizing ultrafine bubbles according to an embodiment of the present invention.
FIG. 2 is a schematic diagram that illustrates the configuration of an apparatus by which the generation of ultrafine bubbles is confirmed.
FIG. 3 is a graph for explaining the state of generation of ultrafine bubbles each time that an aerosol is sprayed.
FIG. 4 is a graph for explaining the generation of ultrafine bubbles by spraying an aerosol.
FIG. 5 is a graph for explaining the generation of ultrafine bubbles by spraying an aerosol.

### [Embodiments of the Invention]

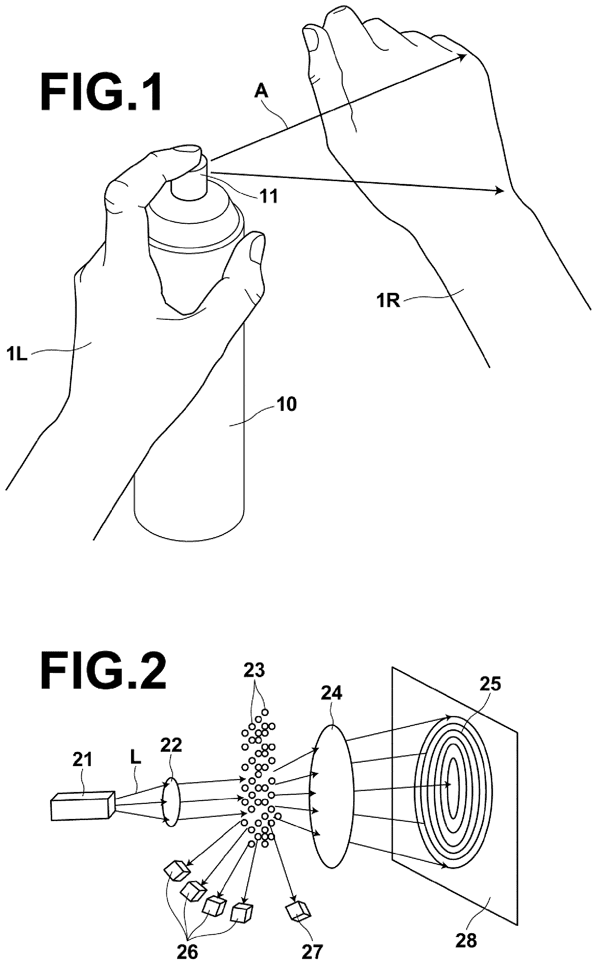

Hereinafter, an embodiment of the present invention will be described with reference to the attached drawings. FIG. 1 is a schematic diagram that illustrates one of the steps of the method for utilizing ultrafine bubbles according to the embodiment of the present invention. In the present embodiment, a cosmetic in which adenosine is blended is applied onto the back of the right hand 1R of a person who wishes to apply the cosmetic, as an example. Prior to the cosmetic being applied, an aerosol container 10 is employed to spray an aerosol spray liquid A to coat the back of the right hand 1R. As an example, aerosol spray liquid A is sprayed by pressing a spray actuator 11 of the aerosol container 10 with a finger of the left hand 1L.

Here, a spray actuator which has a flow rate adjusting mechanism is applied as the spray actuator 11. Specifically, actuator "D391" by K. K. Mitani Valves, which has a flow rate adjusting mechanism is applied in the present embodiment. The detailed configurations of this type of actuator which has a flow rate adjusting mechanism and aerosol containers that employ such actuators are disclosed in Japanese Patent Nos. 4217049, 4350970, and 4974175, for example. In the present embodiment, the spray actuator 11 and the aerosol container 10 can be favorably employed as disclosed in these publications.

That ultrafine bubbles are generated within a liquid in the case that the liquid is sprayed from the spray actuator 11 and the aerosol container 10 was empirically confirmed. The results of the confirmation will be described in detail below. In this experiment, "SALD-7100HH", which is a laser diffraction particle size distribution measuring apparatus produced by K. K. Shimadzu, was utilized to confirm the generation of ultrafine bubbles. The principles of measurement conducted by this laser diffraction particle size distribution measuring apparatus will be described with reference to FIG. 2.

In this apparatus, a laser beam L emitted from a laser light source 21 is collimated by a collimating lens 22, and then is irradiated onto a group of particles 23 which are targets of measurement. In the case of the present example, if a great number of ultrafine bubbles have been generated within the liquid, the ultrafine bubbles will be the group of particles 23. When the laser beam L is irradiated onto the group of particles 23, diffracted/scattered light will be generated toward the back (toward the laser light source 21), the sides, and the front from the group of particles 23. The diffracted/scattered light expresses a predetermined spatial pattern (light intensity distribution pattern) within planes that intersect with the propagation directions thereof. It is known that light intensity distribution patterns are expressed as various shapes depending on the sizes of the particles. Therefore, the percentages of particles and the sizes of particles which are contained (particle size distribution) can be obtained, by detecting and analyzing the light intensity distribution pattern.

In greater detail, this apparatus converges light from the group of particles 23 with a light condensing lens 24 to obtain a diffracted/scattered light image 25. Back scattered light from the group of particles 23 is detected by back scattered light sensors 26, side scattered light from the group of particles 23 is detected by side scattered light sensors 27, and front scattered light from the group of particles 23 is detected by front scattered light sensors 28, and the light intensities of light scattered in each of the directions were obtained. Note that in FIG. 2, the front scattered light sensors 28 are illustrated as a single surface. However, a great number of sensors are provided according to a predetermined arrangement pattern within the surface in actuality. A great number of the rear scattered light sensors 26 and side scattered sensors 27 are arranged in a similar manner. These sensors 26 and 27 mainly detect diffracted/scattered light diffracted/scattered by particles having comparatively small particle sizes within the group of particles 23. In contrast, the front scattered light sensor 28 detects diffracted/scattered light diffracted/scattered by particles having comparatively large particle sizes within the group of particles 23.

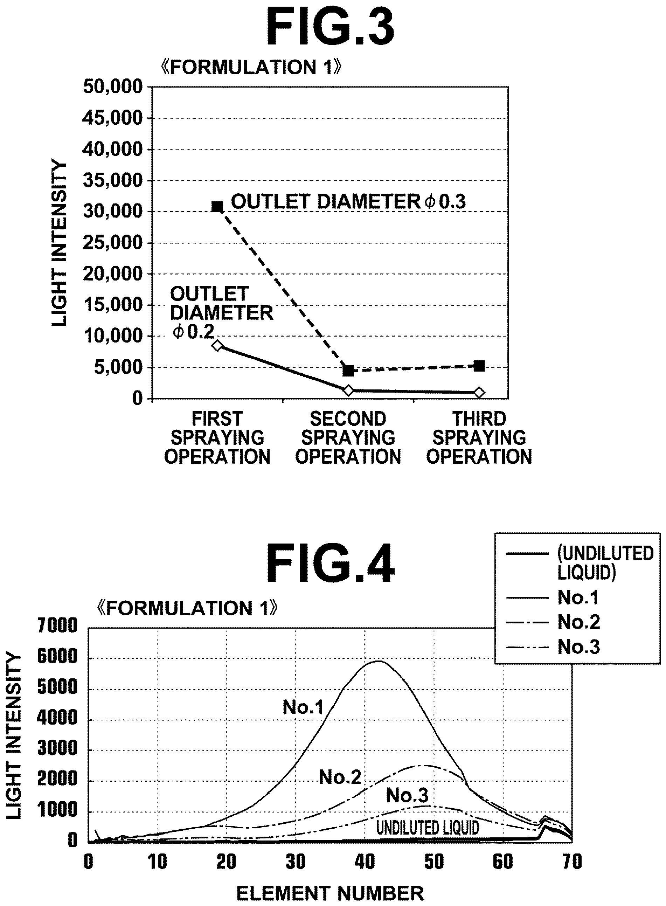

Note that the light intensities which are measured by each of the sensors 26, 27, and 28 will be of higher values as the concentration of the group of particles 23, that is, the ultrafine bubbles, is greater. FIG. 3 illustrates examples of light intensity measurement results in the case that ultrafine bubbles were generated by aerosol spraying employing an actuator having a flow rate adjusting mechanism. In this case, aerosol spraying was conducted a first time, a second time, and a third time, and the light intensities were measured for each instance of aerosol spraying. The horizontal axis in the graph of FIG. 3 represents the instance of aerosol spraying, and the light intensities plotted along the vertical axis indicate relative values, which are differences between an undiluted liquid that does not contain ultrafine bubbles (a liquid which is not sprayed by aerosol) and the aerosol spraying containing ultrafine bubbles.

The curve indicated by the broken line in FIG. 3 represents an example of measurement results for a case in which the actuator "D391" having a flow rate adjusting mechanism was employed with an outlet diameter ϕ of 0.3, and the curve indicated by the solid line represents an example of measurement results for a case in which the actuator "D391" was employed with an outlet diameter ϕ of 0.2. In both cases, measurements were conducted with the aerosol container 10 containing a Formulation 1 to be described later and nitrogen gas as a spraying agent. As illustrated in FIG. 3, it was confirmed that a sufficient amount of ultrafine bubbles were contained in the second and subsequent spraying operations employing both of the actuators. Note that although not illustrated in FIG. 3, in cases that a actuator other than a actuator having a flow rate adjusting mechanism (a single mechanical break up actuator and a double mechanical break up actuator) it was confirmed that ultrafine bubbles were not contained in liquids in second and subsequent spraying operations, and that an effective ultrafine bubble concentration was not obtained.

The light intensities indicated by the sensors 26, 27, and 28 were measured in the manner described above. In the present example, a total of 70 sensors were provided as each of the sensors 26, 27, and 28. Each sensor was designated by a unique element number from "1 through 70", and the light intensities indicated by each of the sensors were measured. The measurement results are shown in FIG. 4. The sensor element numbers along the horizontal axis of the measurement results illustrated in FIG. 4 are the numbers that designate sensors which have detected the intensity distribution patterns of diffracted/scattered light, and establish a correspondent relationship with particle sizes.

There are cases in which the particle size distribution obtained by the laser diffraction/scattering method fluctuate greatly depending on sample conditions, etc. Meanwhile, the local maximum value and the profile of scattered light intensity faithfully reflect the concentration of fine bubbles if the fine bubble generating conditions are the same. Therefore, the local maximum value of scattered light intensity was designated as an index of evaluates the relative concentration of fine bubbles which are contained in the liquid. In both a case in which ultrafine bubbles were generated by aerosol spray and a case in which ultrafine bubbles were generated by employing a pressurized dissolving bubble generator, the scattered light profiles were those having a local maximum in the vicinities of the sensors with element numbers 43 through 45. In addition, element numbers 43 through 45 generally reflect light scattered by particles having particle sizes within a range from 100 to 200 nm that indicate the local maximum value of the number distribution of ultrafine bubbles, regardless of the method by which ultrafine bubbles were generated.

Based on the measurement results of FIG. 4, the light intensity detected by the sensor with element number 43 was extracted, and the concentration of ultrafine bubbles which are relatively contained within the liquid was evaluated. The horizontal axis of the graph illustrated in FIG. 4 is as described above, and vertical axis of the graph illustrated in FIG. 4 indicates the light intensities indicated by each of the sensors as a relative value. The total 70 front scattered light sensors 28 are arranged with the element numbers being smaller toward the center. In other words, the front scattered light sensors 28 with smaller element numbers are arranged closer to direction in which the optical axis extension of the condenser lens 24 illustrated in FIG. 2 extends.

The above light intensity was measured by changing state of the aerosol container 10 in the following four different states, and in each case, Formulation 1 to be described later was sprayed from the aerosol container 10. The above four states are described below. In FIG. 4, measurement results are illustrated with four types of curve corresponding to each of four states. The correspondent relationships between each of the states and each type of curve is shown at the upper right of the graph. No.1 through No.3 are cases in which the type of spray actuator which was applied to the aerosol container 10 were changed. No.1 is a case in which the aforementioned actuator "D391" having the flow rate adjusting mechanism was applied, No. 2 is a case in which a "double mechanical break up actuator" was applied, and No. 3 is a case in which a "single mechanical break up actuator" was applied. In these cases, nitrogen gas was employed as the spraying agent which is filled within the aerosol container 10 along with Formulation 1. In addition, (undiluted liquid) represents a case in which Formulation 1 was applied to the back of the hand without passing through the spray actuator. The measurement results for this case is as indicated in the lowest curve in FIG. 4.

As illustrated in FIG. 4, by the present experiment, it was understood that a light intensity distribution pattern having a local maximum is obtained by the front scattered light sensor 28 in the vicinity of element numbers 43 through 45 in the case that the actuator "D391" having the flow rate adjusting mechanism is employed. It is known in advance that the front scattered light sensor 28 in the vicinity of element numbers 43 through 45 mainly detect diffracted/scattered light from particles having particle diameters within a range from 50 to 1000 nm in the laser diffraction particle size distribution measuring apparatus "SALD-HH" which was utilized in the present experiment. Therefore, it was confirmed that ultrafine bubbles having particle diameters within a range from 50 to 1000 nm were generated.

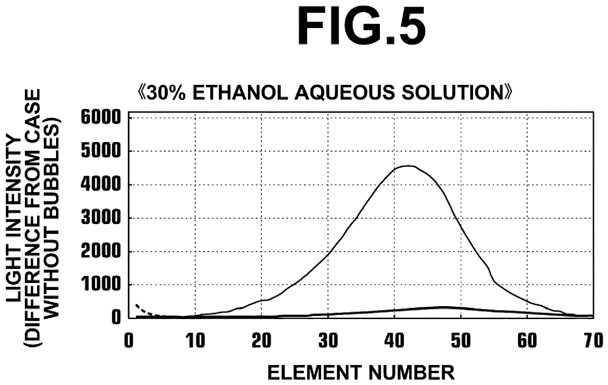

In addition, FIG. 5 shows differences between the light intensities illustrated in FIG. 4 and light intensities which were measured for a 30% ethanol aqueous solution in the same manner. Note that the upper curve in FIG. 5 denotes examples of measurement results for a case in which the aforementioned actuator "D391" having a flow rate adjusting mechanism was employed, and the lower curve denotes examples of measurement results for a case in which the aforementioned "double mechanical break up actuator" was employed. These differences also indicate a light intensity distribution pattern having local maximums at the front scattered light sensor 28 in the vicinities of element numbers 43 through 45. Therefore, it can be understood that the light intensity distribution pattern illustrated in FIG. 4 is that which reflects a concentration distribution of ultrafine bubbles.

Note that the method of utilizing ultrafine bubbles according to the present invention is capable of significantly improving the permeability into the skin of the specific component in the case that the specific component contained in the external skin agent is adenosine. The results of empirical confirmation of this point will be described below. Formulation 1 and a Formulation 2 that contains adenosine were prepared in order to compare the permeabilities thereof. The ingredients of each of the formulations are listed below, and the component ratios are indicated in percentages by mass.

**<<Formulation 1>>**

| | |
|---|---|
| Water | 90% |
| Ethanol | 7.0% |
| DPG | 1.0% |
| DPG-2-deceth-12 | 0.5% |
| Citric Acid | q. s. |
| Sodium Citrate | q. s. |
| Preservative | q. s. |
| Fragrance | q. s. |
| Other Trace Components | 0.3% |

**<<Formulation 2>>**

| | |
|---|---|
| 95% Ethanol | 20.0% |
| Squalene | 1.0% |
| Butylene Glycol | 5.0% |
| Poly Oxyethylene Hardened Castor Oil | 0.2% |
| Glycerin | 5.0% |
| Adenosine | 0.2% |
| Purified Water | Remainder |

In order to provide Formulation 1 that contains ultrafine bubbles onto the skin, the aerosol container 10 was filled with Formulation 1, and the interior pressure was set to 0.9 MPa with nitrogen gas. The actuator "D391" having a flow rate adjusting mechanism produced by K. K. Mitani Valves was applied as the spray actuator 11. In contrast, to provide Formulation 1 that does not contain ultrafine bubbles onto the skin, Formulation 1 was utilized as an undiluted liquid.

That is, a liquid which was sprayed from the aerosol container 10 and Formulation 1 as an undiluted liquid were applied to two different locations on a forearm of a test subject. Thereafter, Formulation 2 that contains adenosine was applied onto each of the two locations for 10 minutes. Next, 1 cm by 1 cm squares of stratum corneum were peeled from the locations onto which the formulations were applied, stratum corneum components were extracted, and the amounts of adenosine were quantitatively analyzed by the LC-MS method (Liquid Chromatography Mass Spectrometry method). The amounts of adenosine within the stratum corneum which were quantitively analyzed indicated that the amount of adenosine was 1.6 times greater in the case that Formulation 1 that contains ultrafine bubbles was applied, compared to the case that Formulation 1 that did not contain ultrafine bubbles was applied. Thereby, the effect of significantly improving the permeability of adenosine due to the utilization of ultrafine bubbles was confirmed.

If Formulation 1 is sprayed from the aerosol container 10 employing the actuator "D391" having the flow rate adjusting mechanism as the spray actuator 11 in the manner described above, the sprayed aerosol liquid becomes a liquid that contains a great amount of fine bubbles having particle diameters of less than or equal to 1000 nm, that is ultrafine bubbles. By applying the sprayed liquid that contains ultrafine bubbles onto the back of the right hand 1R and then applying Formulation 2 thereafter in this manner, an effect that permeation of the specific component within Formulation 2, for example, adenosine, is promoted is obtained. Particularly, in the present embodiment, the sprayed liquid that contains ultrafine bubbles is generated by a simple operation of the aerosol container 10, and therefore application of the liquid that contains ultrafine bubbles is facilitated. Note that the liquid that contains ultrafine bubbles may be produced by a method other than by aerosol spraying.

Note that the sprayed liquid that contains ultrafine bubbles may be directly sprayed and applied from the aerosol container 10 onto a surface where the liquid is utilized (the back of the right hand 1R in the present embodiment) as described above. Alternatively, the sprayed liquid may be sprayed and applied onto a cotton ball for cosmetics or the like, and then the cotton ball for cosmetics or the like may be employed to apply the sprayed liquid onto a utilization surface. Such a manner of application is particularly favorable in the case that the utilization surface of the liquid is the scalp, or areas in the vicinities of the eyes, nose, or ears.

In addition, the method of utilizing ultrafine bubbles according to the present invention is also applicable for a case in which the cosmetic to be applied to human skin is a cosmetic other than Formulation 2, and further for cases in which an external skin agent other than a cosmetic, such as an external pharmaceutical preparation, is to be applied to the skin. In such cases as well, the same effects as those which are described above are exhibited.

Further, the liquid that contains ultrafine bubbles is applied to the skin prior to the external skin agent being applied in the embodiment described above. Alternatively, an external skin agent that contains ultrafine bubbles may be applied to the skin. In this case as well, the same effects can be obtained.

Here, another embodiment of a liquid that contains ultrafine bubbles and is utilizable as the present invention will be described. The liquid constituted by Formulation 1 and Formulation 2 described above will be referred to as Example 1. Other liquids will be described as Examples 2 through 8 below. Table 1 below shows the components of Examples 2 through 4. In the table, the component ratios of each of the components represent percentages by mass with respect to the entirety of the liquid (the same applies hereinafter).

**[Table 1]**

| | Example 2 | Example 3 | Example 4 |
|---|---|---|---|
| Water | 88.2 | 86.3 | 83.8 |
| Glycerin | 1 | 1 | 1 |
| DPG | 5 | 5 | 5 |
| BG | 4 | 4 | 4 |
| PEG/PPG-14/7 Dimethyl Ether | 1 | 1 | |
| PPG-13 Decyl Tetra Deceth-24 | 0.1 | 0.1 | 0.5 |
| Citric Acid | q. s. | q. s. | q. s. |
| Sodium Citrate | q. s. | q. s. | q. s. |
| EDTA | 0.03 | 0.03 | 0.03 |
| Preservative | q. s. | q. s. | q. s. |
| Dipotassium Glycyrrhizinate | 0.07 | | |
| Potassium Salt 4-Methoxy Salicylate | | 2 | |
| Tocopherol Acetate | | | 0.07 |

Next, the components of the liquids of Examples 5 through 8 will be shown below. Each of Examples 5 through 8 are liquids that applies a liquid that contains ultrafine bubbles by spraying Formulation 1 onto the skin and then applying Formulation 2 onto the same location of the skin after Formulation 1 has blended thereinto. That is, Table 2 shows the component ratios of Formulation 1 which is utilized in common in Examples 5 through 8, and Table 3 shows the component ratios of Formulation 2 in each of Examples 5 through 8.

**[Table 2]**

| Formulation 1 | |
|---|---|
| Component | Examples 5 through 8 |
| Water | 90 |
| Ethanol | 7 |
| DPG | 1 |
| DPG-2-deceth-12 | 0.5 |
| Citric Acid | q. s. |
| Sodium Citrate | q. s. |
| EDTA | q. s. |
| Preservative | q. s. |

**[Table 3]**

| Formulation 2 | | | | |
|---|---|---|---|---|
| | Example 5 | Example 6 | Example 7 | Example 8 |
| Water | 58.5 | 58.43 | 56.5 | 58.43 |
| Ethanol | 20 | 20 | 20 | 20 |
| DPG | 15 | 15 | 15 | 15 |
| Sorbitol | 4 | 4 | 4 | 4 |
| Octyl Dodeceth-16 | 0.2 | 0.2 | 0.2 | 0.2 |
| EDTA | 0.02 | 0.02 | 0.02 | 0.02 |
| (Dimethyl Acrylamide/Acryloyl Dimethyl Taurine Na) Cross Polymer | 0.72 | 0.72 | 0.72 | 0.72 |
| Fragrance | q. s. | q. s. | q. s. | q. s. |
| Other Trace Components | 0.55 | 0.55 | 0.55 | 0.55 |
| Adenosine | 0.55 | 0.55 | 0.55 | 0.55 |
| Dipotassium Glycyrrhizinate | | 0.07 | | |
| Potassium Salt 4-Methoxy Salicylate | | | 2 | |
| Tocopherol Acetate | | | | 0.07 |

Note that the effective component which is contained in the external skin agent as the specific component may be constituted by a lipophilic compound or may be constituted by a hydrophilic compound. In the case that a permeation promoting agent is utilized with a lipophilic compound, for example, it is generally difficult to blend a component that also improves the permeability of a hydrophilic compound as well in the same type of preparation. The method for utilizing ultrafine bubbles according to the present invention employs ultrafine bubbles, which have a low degree of correlation with chemical properties. Therefore, it is possible to efficiently promote the permeation of all of a plurality of effective components. The method of utilizing ultrafine bubbles according to the present invention is applicable to a greater range of external skin agents compared to cases in which permeation promoting agents are utilized.

### [Explanation of the Reference Numerals]

- 1L: left hand
- 1R: right hand
- 10: aerosol container
- 11: spray actuator having flow rate adjusting mechanism
- 21: laser light source
- 22: collimating lens
- 23: group of particles
- 24: light condensing lens
- 25: diffracted/scattered image
- 26: back scattered light sensors
- 27: side scattered light sensors
- 28: front scattered light sensors

## Claims

1. A method of utilizing ultrafine bubbles, comprising one of:
applying a liquid that contains ultrafine bubbles onto a portion of the skin of a human body onto which an external skin agent that contains a specific component is to be applied to the skin; and
applying an external skin agent that contains ultrafine bubbles onto the skin;
in order to promote permeation of the specific component into the skin.

2. The method of utilizing ultrafine bubbles according to Claim 1, wherein:
the specific component is adenosine.

3. The method of utilizing ultrafine bubbles according to either one of Claims 1 and 2, wherein:
a principal component of the liquid is water.

4. The method of utilizing bubbles according to any one of Claims 1 through 3, wherein:
the external skin agent is one of a cosmetic and an external pharmaceutical preparation.
